# EUROPEAN PATENT APPLICATION

(11) **EP 1 336 601 A1**
(43) Date of publication of application: **20.08.2003**
(21) Application number: 01982860.7
(22) Date of filing: 20.11.2001
(51) Int. Cl.: C07C 67/287, C07C 69/708, C07C 51/09, C07C 59/135, G01N 30/88, G01N 30/86, G01N 30/04

(54) **PROCESS FOR PREPARING FLUORINATED ESTERS BY GAS CHROMATOGRAPHY**

(30) Priority: 20.11.2000 JP 2000353340
(71) Applicant: ASAHI GLASS COMPANY LTD., Tokyo 100-8405 (JP)
(72) Inventor: KAWAHARA, Kengo, Asahi Glass Company, Limited, Yokohama-shi, Kanagawa 221-8755 (JP); ISEMURA, Tsuguhide, Asahi Glass Company, Limited, Ichihara-shi, Chiba 290-8566 (JP); OKAZOE, Takashi, Asahi Glass Company, Limited, Yokohama-shi, Kanagawa 221-8755 (JP)
(74) Representative: Müller-Boré & Partner Patentanwälte
(86) International application number: JP0110116
(87) International publication number: WO02040437

(57) **Abstract**

The present invention provides a production method to effectively obtain a product in a yield equal to or higher than an objective level, by accurately analyzing the amount of a fluorinated ester compound at the time of its production, or by accurately analyzing the consumption amount or the produced amount of the product or the fluorinated ester compound at the time of chemical conversion of the fluorinated ester compound.

Namely, it is the method for producing a fluorinated ester compound by a chemical reaction of a raw material compound, wherein the chemical reaction is carried out until the yield of the fluorinated ester compound as obtained by gas chromatography by using a non-polar column reaches a predetermined yield.

## Description

### TECHNICAL FIELD

The present invention relates to a method for producing a fluorinated ester compound by means of gas chromatography (hereinafter referred to as GC method), a method for producing a chemically converted product by chemical conversion of a fluorinated ester compound employing GC method, and a method for analyzing a fluorinated ester compound. The method of the present invention is useful as a method for forming or a method of chemically converting a fluorinated ester compound with a predetermined reaction result, a quality control method for a fluorinated ester compound, and the like.

### BACKGROUND ART

In a case where a fluorinated ester compound is produced, or in a case where a fluorinated ester compound is chemically converted to obtain a product, quantitative analysis and/or qualitative analysis (hereinafter quantitative analysis and/or qualitative analysis will be referred to simply as analysis) of the fluorinated ester compound is important in a case of confirming the progress of the reaction, in a case of controlling the production conditions, in a case of controlling the quality of the obtained compound, and the like.

Heretofore, GC method has been known as a method for analyzing a fluorinated compound. Further, as a method of injecting a sample in the GC method, a split method, a splitless method, a moving needle method, a cryofocus method and the like have been known.

Further, as another analysis method, nuclear magnetic resonance (NMR method), high performance liquid chromatography (HPLC method), supercritical fluid liquid chromatography (SFC method), a combination of HPLC method or SFC method with mass spectrometry (MS method) and the like have been known.

However, with conventional GC method, the analysis tends to be difficult when the fluorinated compound is thermally unstable. Even when the fluorinated ester compound is a chemically stable compound, if it is analyzed by conventional GC method, dissociation of the compound may take place, and the fluorinated ester compound cannot directly be analyzed, such being problematic.

Further, NMR method is a general purpose method, however, the analysis operation is complicated, and as no separation is carried out in the method, adequate quantitative determination tends to be difficult, and the sensitivity tends to be low, such being problematic. Further, HPLC method and SFC method have such a problem that the resolution and the sensitivity tend to be low.

### DISCLOSURE OF THE INVENTION

It is an object of the present invention to provide a production method to effectively obtain a product in a yield equal to or higher than an objective level, and a method for analyzing a fluorinated ester compound useful for e.g. quality control of said compound, by accurately analyzing the consumed amount or the formed amount of the fluorinated ester compound, in a method for effectively producing a fluorinated ester compound and a production method of chemically converting a fluorinated ester compound to obtain a product, by accurately analyzing the fluorinated ester compound.

Namely, the present invention provides a method for producing a fluorinated ester compound by a chemical reaction of a raw material compound, wherein the chemical reaction is carried out until the yield of the fluorinated ester compound as obtained by GC method by using a non-polar column reaches is a predetermined yield.

The present invention further provides a method of chemically converting a fluorinated ester compound to obtain a product, wherein the chemical conversion is carried out until the degree of conversion of the fluorinated ester compound as obtained by GC method by using a non-polar column reaches a predetermined degree of conversion.

The present invention still further provides a method for analyzing a fluorinated ester compound, which comprises analyzing a fluorinated ester compound contained in a sample containing the fluorinated ester compound by GC method by using a non-polar column.

### BRIEF DESCRIPTION OF THE DRAWINGS

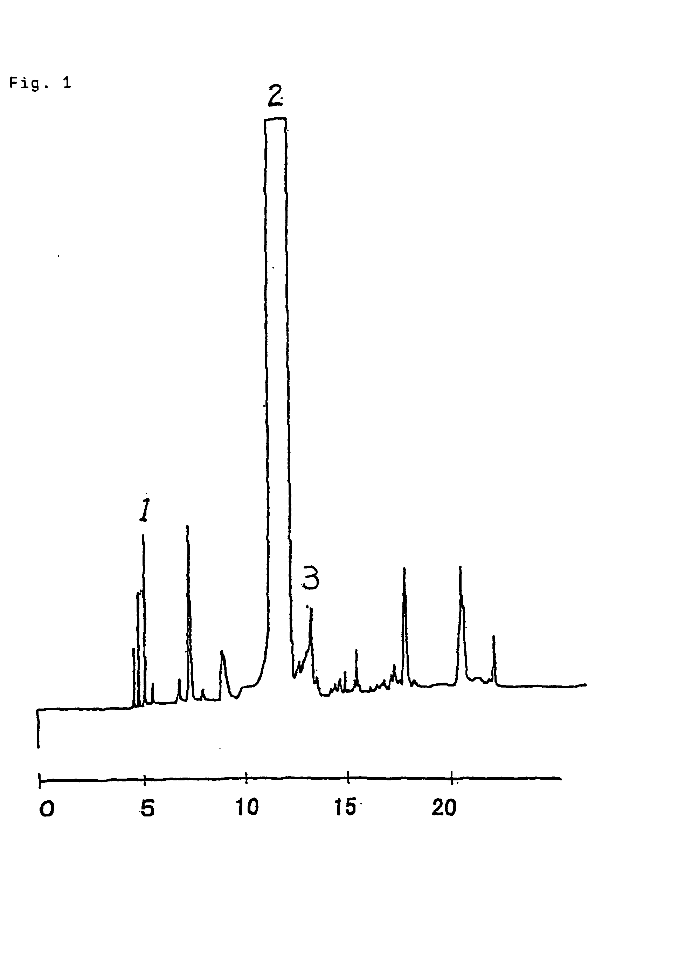

Fig. 1 is a gas chromatogram (horizontal axis represents time (minute)) of a fluorinated ester compound obtained by a fluorination reaction according to Analysis Example 1.

In Fig. 1, the numerical reference 1 designates a peak of perfluoroacyl fluoride, the numerical reference 2 designates a peak of perfluoroester, and the numerical reference 3 designates a peak of partially fluorinated ester.

### BEST MODE FOR CARRYING OUT THE INVENTION

The fluorinated ester compound in the present invention is a compound having a fluorine atom and an ester bond. The compound is preferably a compound having a partial structure of the following formula 1 wherein a fluorine atom is bonded to a carbon atom adjacent to the oxygen atom in the ester bond:

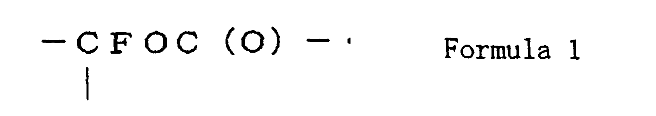

In the formula 1, to each of two bonds from a carbon atom to which a fluorine atom is bonded, a monovalent organic group or a monovalent atom may be bonded, and the two bonds may form a double bond. Further, the carbon atom to which a fluorine atom is bonded may be a carbon atom which forms a ring. It is preferred that a fluorine atom or a monovalent organic group is bonded to each of the two bonds from the carbon atom to which the fluorine atom is bonded in the formula 1, and it is particularly preferred that a monovalent organic group is bonded to at least one bond. The compound having such a partial structure in its molecule is a compound which may undergo dissociation reaction of the ester bond, and is a compound which has been difficult to directly analyze by the conventional GC method, as it undergoes dissociation by the influence of heat at an inlet or the column. On the other hand, the fluorinated ester compound is preferably a compound having a boiling point of at most 400°C in view of easiness of GC analysis.

In the present invention, a specific GC method is employed in the method for producing a fluorinated ester compound by a chemical reaction of a raw material compound. The production method may, for example, be a method for producing a fluorinated ester compound by a fluorination reaction of a raw material compound which can be fluorinated, a method of chemically converting a partial structure of a fluorinated ester compound to obtain another fluorinated ester compound -(such as a method of exchanging functional groups), or a method of reacting a carboxylic acid with an alcohol (provided that at least one of the carboxylic acid and the alcohol is a fluorine-containing compound) to obtain a fluorinated ester compound.

Among these production methods, it is particularly advantageous to apply the method of the present invention to the method for producing a fluorinated ester compound by a fluorination reaction of a raw material compound which can be fluorinated, since a plurality of compounds are formed by the reaction, and separation and analysis will be required.

The fluorination reaction may, for example, be a method of fluorinating a chlorine atom with potassium fluoride to replace the chlorine atom by a fluorine atom, a method of fluorinating a hydrogen atom to replace it by a fluorine atom (hereinafter referred to as direct fluorination) or a method of reacting fluorine with an unsaturated bond. Now, a method for producing a fluorinated ester compound by direct fluorination will be explained as a representative example.

The raw material compound in direct fluorination is preferably an ester compound having a hydrogen atom. Further, direct fluorination may, for example, be a method of reaction with fluorine (F₂) in a liquid phase (JP-A-4-500520, WO00/56694), cobalt fluorination method or electrochemical fluorination method, and a method of reaction with fluorine in a liquid phase is preferred. By direct fluorination, a hydrogen atom is replaced by a fluorine atom, and in a case where an unsaturated bond is present between carbon atoms of the compound, fluorine atoms are added to the unsaturated bond to form a fluorinated ester compound.

The rate of fluorine introduction of the fluorinated ester compound (the rate of fluorine introduction is a proportion of number of fluorine atoms in the fluorinated ester compound to the total number of fluorine atoms in a perfluorinated ester compound formed by completely fluorinating a raw material compound which can be fluorinated) is a predetermined value depending upon the intended compound, and the predetermined value in a usual reaction is set as a value equal to or higher than a specific value. The predetermined value is preferably at least 80%, particularly preferably at least 98%, and especially preferably substantially 100% in a usual case. Particularly the rate of fluorine introduction in direct fluorination is preferably 100%.

In the present invention, the fluorinated ester compound is analyzed by GC method by using a non-polar column. By GC method by using a non-polar column, an accurate analysis can be carried out by favorably separating the fluorinated ester compound from other compounds without dissociation of the fluorinated ester compound. Further, by occasionally analyzing the fluorinated ester compound at the time of production of the fluorinated ester compound, reaction conditions for effective production of the compound can be controlled, and the end point of the reaction can be determined.

As the column used for GC method, a non-polar column which does not dissociate the fluorinated ester compound is employed. The non-polar column is preferably a column having a layer comprising 100% poly(dimethylsiloxane) in the liquid phase or on the inner surface of the column, particularly preferably a Wall Coated Open Tubular (WCOT type) non-polar type column. By using such a column, the fluorinated ester compound can be analyzed without substantially dissociating the fluorinated ester compound.

Further, the non-polar column is preferably a capillary column in view of resolution, particularly preferably a capillary column having a length of from 5 to 120 m, especially preferably a capillary column having a length of from 30 to 60 m form such reasons as general purpose elution time, number of theoretical plate, degree of separation and convenience. Further, the inner diameter of the capillary column is not particularly limited, and is preferably from 0.2 to 1.2 mm. The thickness of the liquid phase of the capillary column is preferably from 0.1 to 5 µm, particularly preferably from 0.25 to 1.0 µm

Further, with a purpose of solvent effect or to trap nonvolatile components, a precolumn may be connected to the inlet side of the non-polar column by means of a column connector. The precolumn is preferably one having a length of from 5 cm to 10 m, and it is particularly preferred to employ one having a length at a level of 1 m. The inner diameter of the precolumn is preferably from 0.1 to 1 mm, and the inner diameter is advisably from 0.25 to 0.53 mm since a needle made of stainless steel or a needle made of fused silica is employed. Further, as the precolumn, it is preferred to employ a deactivated fused silica hollow capillary.

In GC method for the fluorinated ester compound, it is preferred to employ cool on-column injection method or programmed temperature-raising vaporization method (hereinafter referred to as PTV method) as the injection method, and cool on-column method is particularly preferred, which is for general purposes and which can introduce a sample to the column without vaporizing it. The cool on-column injection method is a known method, and is generally useful for thermally unstable compounds. Further, by the cool on-column injection method, the whole amount of the sample can be introduced to the column, whereby selective vaporization or selective column introduction of the sample components to be analyzed is less likely to take place at the syringe or the vaporization portion, and thus it is a method excellent in quantitative analysis property and reproducibility.

Further, it is particularly preferred to carry out the cool on-column method by means of an autoinjector, whereby factitious sampling errors can be avoided, and quantitative determination can be performed with higher accuracy and precision. The inlet for the cool on-column method is commercially available, and on-column inlets manufactured by Agilent Technologies, Perkin Elmer Inc. and Shimadzu Corporation may, for example, be mentioned.

For a detector in GC method, a detector of any principle may be employed. For example, a flame ionization detector, a thermal conductivity detector, a nitrogen/phosphorus detector, an electron capture detector, a flame photometry detector, a photo ionization detector, an electric conductivity detector, a surface ionization detector, a chemiluminescence detector, a mass spectrometer, a microwave induced plasma emission detector, a Fourier transform infrared spectrophotometer or a chemiluminescence detector may be employed. Among them, a mass spectrometer and a flame ionization detector which are commonly used for analysis of organic compounds such as a fluorinated ester compound are preferred. To the analysis of the fluorinated ester compound, quantitative analysis method or qualitative analysis method in conventional GC analysis can be directly applied.

In the present invention, in the method for producing a fluorinated ester compound, the chemical reaction is carried out until the yield of the fluorinated ester compound as obtained by the above GC method reaches a predetermined yield. Said predetermined yield is optional, and is usually set to a value equal to or higher than a specific yield, and may optionally be changed depending upon the reaction to be carried out.

For example, in a method for producing a fluorinated ester compound by reacting an ester compound having hydrogen atoms with fluorine, an intended compound can be obtained in a predetermined yield by analyzing the intended compound having a predetermined rate of fluorine introduction by GC method. For example, in order to obtain an intended compound having a rate of fluorine introduction of substantially 100%, the reaction conditions can be controlled by analyzing the intended compound contained in the reaction crude liquid during the fluorination reaction, whereby the end point of the reaction can be known, and the intended compound can be obtained in a predetermined yield. As the fluorinated ester compound, an ester compound having substantially 100% of hydrogen atoms fluorinated is particularly preferred in view of usefulness.

Further, in a case where direct fluorination is carried out in a liquid phase, irradiation with ultraviolet rays or benzene injection may be carried out in some cases at the end of the reaction to increase the rate of fluorine introduction. In this case also, the timing of the irradiation with ultraviolet rays or the benzene injection can be determined by analyzing the amount of the fluorinated ester having a predetermined rate of fluorine introduction. Further, by analyzing the amount of the fluorinated ester having a predetermined rate of fluorine introduction after irradiation with ultraviolet rays or after the benzene injection, the end point of the reaction can be determined.

Further, according to the present invention, in the method of chemically converting a fluorinated ester compound to produce a product by analyzing the fluorinated ester compound by the same GC method as mentioned above, production can be carried out wherein the degree of conversion of the fluorinated ester compound is at a predetermined degree of conversion. The predetermined degree of conversion may also be set to a value equal to or higher than a specific value in a usual case.

As specific examples of the chemical conversion of the fluorinated ester compound, a method of obtaining an acyl fluoride compound or a ketone compound from a product of a dissociation reaction wherein an ester dissociation reaction of the fluorinated ester compound is carried out, a method of heating the fluorinated ester compound to convert it into a compound containing a vinyl fluoride group and containing no ester bond, and a method of carrying out transesterification of the fluorinated ester compound may, for example, be mentioned. In said chemical conversion, by analyzing the amount of the fluorinated ester compound contained in the reaction crude liquid, the degree of conversion of the fluorinated ester compound is obtained. Further, by occasionally knowing the degree of conversion in the reaction crude liquid, effective chemical conversion can be carried out.

Further, in a case where GC method is carried out under GC analysis conditions under which analysis of the product is possible, in chemical conversion of the fluorinated ester compound, the yield of the product can also be analyzed. For example, in a case where ester dissociation reaction of the fluorinated ester compound is carried out to obtain an acyl fluoride compound from the product of the dissociation reaction, both the yield of acyl fluoride and the degree of conversion of the fluorinated ester compound can optionally be known by a single analysis, and the reaction conditions for effective production of the product can be controlled to determine the end point of the chemical conversion.

The present invention further provides a method for analyzing a fluorinated ester compound contained in a sample by GC method by using a non-polar column. The method is particularly useful as an analysis method for quality control of the fluorinated ester compound. The sample in the method may, for example, be a reaction crude product containing the fluorinated ester compound obtained by a chemical reaction of a raw material compound, a fluorinated ester compound obtained by purifying said product, a reaction crude product containing a product obtained by chemical conversion of a fluorinated ester compound, or a product obtained by purifying said reaction crude product.

### EXAMPLES

Now, the present invention will be specifically explained with reference to Examples and Comparative Examples. However, the present invention is by no means restricted to such explanations.

### Analysis conditions

Using as a main column, a non-polar capillary column (manufactured by J&W, DB-1, length: 60 m, inner diameter: 0.25 mm, thickness: 1.0 µm) , and as a precolumn, a deactivated fused silica hollow capillary (manufactured by GL Sciences Inc., fused silica capillary deactivated tube, length: 1 m, inner diameter: 0.530 mm, outer diameter: 0.660 mm), they were connected by means of a capillary column connector (manufactured by GL Sciences Inc., Pressfit Universal Union).

The oven temperature was maintained at 20°C for 5 minutes, then the temperature was raised from 20°C to 270°C at a rate of 10°C/min, and the temperature was maintained at 270°C for 5 minutes. A flame ionization detector was employed as the detector. For determination of the structure of a compound corresponding to each peak on the chromatogram, a mass spectrometer was employed as the detector. Helium gas was employed as the carrier gas, the temperature of the detector was 280°C, the amount of sample introduction was 0.03 µL, and the column top pressure was set so that the column linear velocity would be 25 cm/min.

### Analysis Example 1

NaF was put in a 500 mL autoclave made of nickel, 1,1,2-trichlorotrifluoroethane (R-113) was added and stirred, followed by cooling to -10°C. After blowing nitrogen gas for 1 hour, fluorine gas diluted to 20% with nitrogen gas was blown for 1 hour, and a solution having H(CH₂)₃OCH(CH₃)CH₂OC(O)CF(CF₃)O(CF₂)₃F dissolved in R-113 was injected over a period of 19.4 hours.

Then, while blowing fluorine gas diluted to 20% with nitrogen gas, an R-113 solution of benzene was injected, the outlet valve of the autoclave was closed, and the inlet valve of the autoclave was closed when the pressure became 0.12 MPa, and stirring was continued for 1 hour. This operation was carried out four times while the temperature was raised from -10°C to room temperature, and then carried out five times at room temperature. Then, nitrogen gas was blown for 2 hours, and the reaction mixture was taken out by decantation to obtain a sample 1.

The gas chromatogram obtained by GC analysis of the sample 1 under the above analysis conditions is shown in Fig. 1. It was confirmed that on the gas chromatogram, peaks of F (CF₂)₃OCF(CF₃)CF₂OC(O)CF(CF₃-)O(CF₂)₃F (hereinafter referred to as perfluoroester), partially fluorinated ester and F(CF₂)₃OCF(CF₃)COF (hereinafter referred to as perfluoroacyl fluoride) were completely separated.

Further, after a yield of perfluoroester (rate of fluorine introduction was 100%) of 95% in the gas chromatogram was confirmed, the reaction was completed. The amount of perfluoroacyl fluoride contained in the sample was 0.6 mol% (amount based on perfluoroester). The same sample 1 was analyzed by NMR method and as a result, the amount of perfluoroacyl fluoride was 0.4 mol% (amount based on perfluoroester). Namely, it was confirmed that analysis can be carried out substantially without dissociation of perfluoroester.

### Analysis Example 2

The above-obtained sample 1 was heated to 145 °C in the presence of NaF. The reaction crude liquid during the reaction was subjected to GC analysis under the same analysis conditions as mentioned above. As a result, perfluoroester and perfluoroacyl fluoride were detected in the reaction crude liquid, but the amount of perfluoroester decreased along the progress of the reaction. The reaction was completed when substantially no peak of perfluoroester was observed on the gas chromatogram. The degree of conversion of perfluoroester was 98%.

Further, on the gas chromatogram, no peak of F(CF₂)₃OCF=CF₂ as a pyrolysis product of perfluoroacyl fluoride was detected. The reaction crude liquid after completion of the reaction was further subjected to NMR analysis, and the result agreed with the result by GC analysis.

### INDUSTRIAL APPLICABILITY

According to the production method of the present invention, production of a fluorinated ester compound and chemical conversion of a fluorinated ester compound can effectively be carried out while accurately analyzing the amount of the fluorinated ester compound. Namely, production can be carried out while the degree of progress of the fluorination reaction or pyrolysis is accurately determined. Further, the analysis method of the present invention is useful also as a method for controlling steps in production employing a fluorinated ester compound. Further, the analysis method of the present invention is advantageous also as a method of quality control of products.

## Claims

1. A method for producing, a fluorinated ester compound by a chemical reaction of a raw material compound, wherein the chemical reaction is carried out until the yield of the fluorinated ester compound as obtained by gas chromatography by using a non-polar column reaches a predetermined yield.

2. The production method according to Claim 1, wherein the chemical reaction of a raw material compound is a reaction of fluorinating a raw material compound which can be fluorinated to obtain a fluorinated ester compound.

3. The production method according to Claim 1 or 2, wherein the following rate of fluorine introduction of the fluorinated ester compound is at a predetermined rate of introduction:
rate of fluorine introduction: proportion of the number of fluorine atoms in the fluorinated ester compound, to the total number of fluorine atoms in a perfluorinated ester compound formed by completely fluorinating a raw material compound which can be fluorinated.

4. The production method according to Claim 3, wherein the above rate of fluorination introduction of the fluorinated ester compound is substantially 100%.

5. The production method according to Claim 2, 3 or 4, wherein the fluorination is carried out by reaction with fluorine in a liquid phase.

6. The production method according to Claim 5, wherein for the fluorination, irradiation with ultraviolet rays or benzene injection is carried out.

7. A method of chemically converting a fluorinated ester compound to obtain a product, wherein the chemical conversion is carried out until the degree of conversion of the fluorinated ester compound as obtained by gas chromatography by using a non-polar column reaches a predetermined degree of conversion.

8. The production method according to Claim 7, wherein the selection rate of the fluorinated ester compound is obtained by gas chromatography by using a non-polar column.

9. The production method according to Claim 7 or 8, wherein the method of chemically converting a fluorinated ester compound to obtain a product is a method of pyrolyzing the fluorinated ester compound to obtain a dissociation reaction product.

10. The production method according to any one of Claims 1 to 9, wherein as the injection method in the gas chromatography, cool on-column injection method is employed.

11. The production method according to any one of Claims 1 to 10, wherein the fluorinated ester compound is a compound having a structure of the following formula 1 as a partial structure:

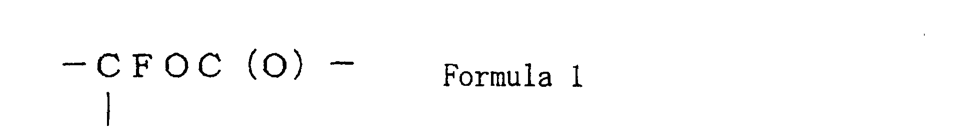

12. A method for analyzing a fluorinated ester compound, which comprises analyzing a fluorinated ester compound contained in a sample containing the fluorinated ester compound by gas chromatography by using a non-polar column.

13. The analysis method according to Claim 12, wherein the fluorinated ester compound is a compound having a structure of the following formula 1 as a partial structure:
